# EUROPÄISCHE PATENTSCHRIFT

(11) **EP 0 716 081 B1**
(45) Veröffentlichungstag und Bekanntmachung des Hinweises auf die Patenterteilung: **05.04.2006**
(21) Anmeldenummer: 95118359.9
(22) Anmeldetag: 22.11.1995
(51) Int. Cl.: C07D 211/90, C07D 207/40, C07D 401/12, A61K 31/44

(54) **Hochselektives Verfahren zur Herstellung von enantiomerenreinen phenylsubstituierten 1,4-Dihydropyridin-3,5-dicarbonsäurederivaten**
Highly selective process for the preparation of enantiomerically pure phenyl-substituted 1,4-dihydropyridine-3,5-dicarboxylic acid derivatives
Procédé à sélectivité élevée pour la préparation de dérivés d'acide 1,4-dihydropyridine-3,5-dicarboxylique phénylsubstitué énantiomériquement purs

(30) Priorität: 05.12.1994 DE 4443168
(43) Veröffentlichungstag der Anmeldung: 12.06.1996
(60) Teilanmeldung: 04009787.5
(73) Patentinhaber: Bayer HealthCare AG, 51368 Leverkusen (DE)
(72) Erfinder: Mittendorf, Joachim, Dr., D-42113 Wuppertal (DE); Fey, Peter, Dr., D-42111 Wuppertal (DE); Junge, Bodo, Dr., D-42399 Wuppertal (DE); Kaulen, Johannes, Dr., D-37691 Boffzen (DE); Van Laak, Kai, Dr., D-51065 Köln (DE); Meier, Heinrich, Dr., Tsurumai Nishimashi, Nara 631 (JP); Schohe-Loop, Rudolf, Dr., D-42327 Wuppertal (DE)

(56) Entgegenhaltungen:
- EP-A- 0 160 451
- EP-A- 0 166 296
- ANGEW. CHEM., Bd. 103, Nr. 7, 1991 Seiten 1587-1605, S. GOLDMANN ET AL

## Beschreibung

Die vorliegende Erfindung betrifft ein neues selektives Verfahren zur Herstellung von enantiomerenreinen phenylsubstituierten 1,4-Dihydropyridin-3,5-dicarbonsäurederivaten.

Die Publikation Angew. Chem. 103, 1991, 1587-1605 beschreibt die große Bedeutung der absoluten Stereochemie von 1,4-Dihydropyridinen für ihre pharmakologische Wirkung als Calciumantagonisten oder -agonisten und eine Aufstellung der heute verfügbaren Methoden zu ihrer Herstellung in enantiomerenreiner Form. Schwerpunkt aller dieser Verfahren ist die Trennung diastereomerer Ester mit Chiralpool Auxiliaren, wobei deren Auswahl im Hinblick auf die oft auftretenden großen Schwierigkeiten bei deren Herstellung und der Einführung und Abspaltung in bzw. aus den Molekülen letztendlich einem trial- und error-Verfahren gleicht. Insbesondere die Abspaltung vieler Auxiliare erfordert oft einen hohen technischen und chemischen Aufwand, der wiederum mit einer Verminderung der Ausbeute verbunden ist.

Überraschenderweise wurde nun eine hochselektive Methode durch Einsatz von Äpfelsäureimiden als Auxiliare gefunden.

Gegenstand der Erfindung ist ein neues hochselektives Verfahren zur Herstellung von enantiomerenreinen phenylsubstituierten 1,4-Dihydropyridin-3,5-dicarbonsäurederivaten der allgemeinen Formel (I), 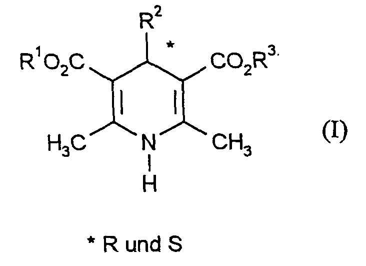 in welcher
- R¹ und R³: gleich oder verschieden sind und für geradkettiges oder verzweigtes Alkyl mit bis zu 8 Kohlenstoffatomen stehen, das gegebenenfalls durch geradkettiges oder verzweigtes Alkoxy mit bis zu 6 Kohlenstoffatomen oder Hydroxy substituiert ist, oder
für Cycloalkyl mit 3 bis 7 Kohlenstoffatomen stehen,
und
- R²: für den Rest 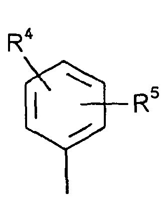 steht, worin
R⁴ und R⁵ gleich oder verschieden sind und
Halogen, Cyano, Ethinyl, Trifluormethoxy, Methylthio, Nitro, Trifluormethyl oder geradkettiges oder verzweigtes Alkyl, Alkenyl, Alkinyl oder Alkoxy bis zu 4 Kohlenstoffatomen bedeuten, und gegebenenfalls einer der Substituenten für Wasserstoff steht,
und deren Salze,
dadurch gekennzeichnet, daß man die enantiomerenreinen Benzylidenverbindungen der allgemeinen Formel (II) oder die Benzylidenverbindungen der allgemeinen Formel (IIa) 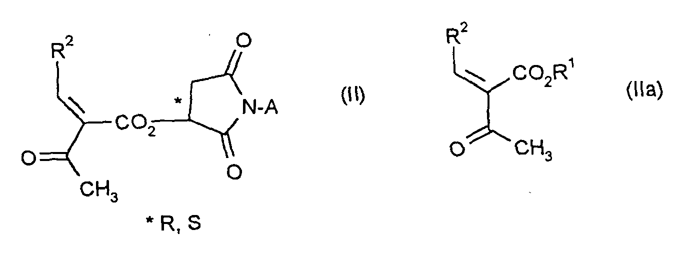 in welchen
- R¹ und R²: die oben angegebenen Bedeutungen haben
und
- A: für Wasserstoff oder für geradkettiges oder verzweigtes Alkyl mit bis zu 8 Kohlenstoffatomem steht, oder
für Phenyl oder Benzyl steht, die gegebenenfalls bis zu 3-fach gleich oder verschieden durch Hydroxy, Nitro, Halogen, Cyano, Carboxy, Trifluormethyl, Trifluormethoxy, geradkettiges oder verzweigtes Alkoxy mit bis zu 6 Kohlenstoffatomen oder durch eine Gruppe der Formel -NR⁶R⁷ oder -SO₂R⁸ substituiert sind,
worin
R⁶ und R⁷ gleich oder verschieden sind und
Wasserstoff, Phenyl oder geradkettiges oder verzweigtes Alkyl mit bis zu 5 Kohlenstoffatomen bedeuten und
R⁸ geradkettiges oder verzweigtes Alkyl mit bis zu 4 Kohlenstoffatomen oder Phenyl bedeutet,
durch Umsetzung,
im Fall der enantiomerenreinen Benzylidenverbindungen der allgemeinen Formel (II) mit Aminocrotonsäureestern der allgemeinen Formel (III) und im Fall der Benzylidenverbindungen der allgemeinen Formel (IIa) mit den entsprechenden enantiomerenreinen Aminocrotonsäureestern der allgemeinen Formel (IIIa) 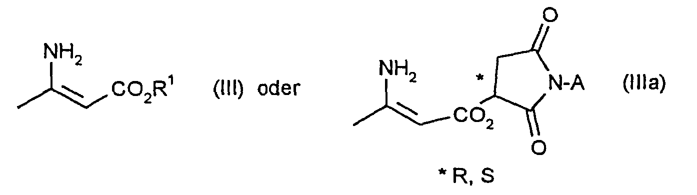 in welchen
R¹ und A die oben angegebenen Bedeutungen haben,
in inerten Lösemitteln, gegebenenfalls in Anwesenheit einer Base,
in die diastereomerenreinen 1,4-Dihydropyridine der allgemeinen Formeln (IVa) und (IVb) 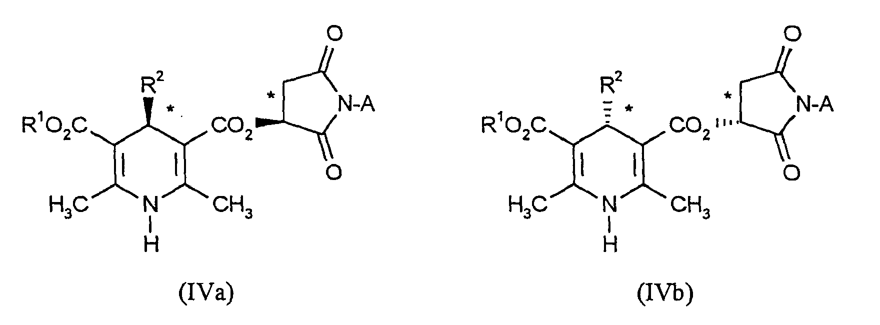 in welcher
R¹, R² und A die oben angegebenen Bedeutungen haben,
überführt,
und anschließend den Äpfelsäureimid-Rest unter milden Bedingungen mit schwachen Basen, gegebenenfalls unter Isolierung der freien Säure, abspaltet, und die Carbonsäurefunktion nach üblichen Methoden verestert.

Das erfindungsgemäße Verfahren kann durch folgende Formelschema beispielhaft erläutert werden: 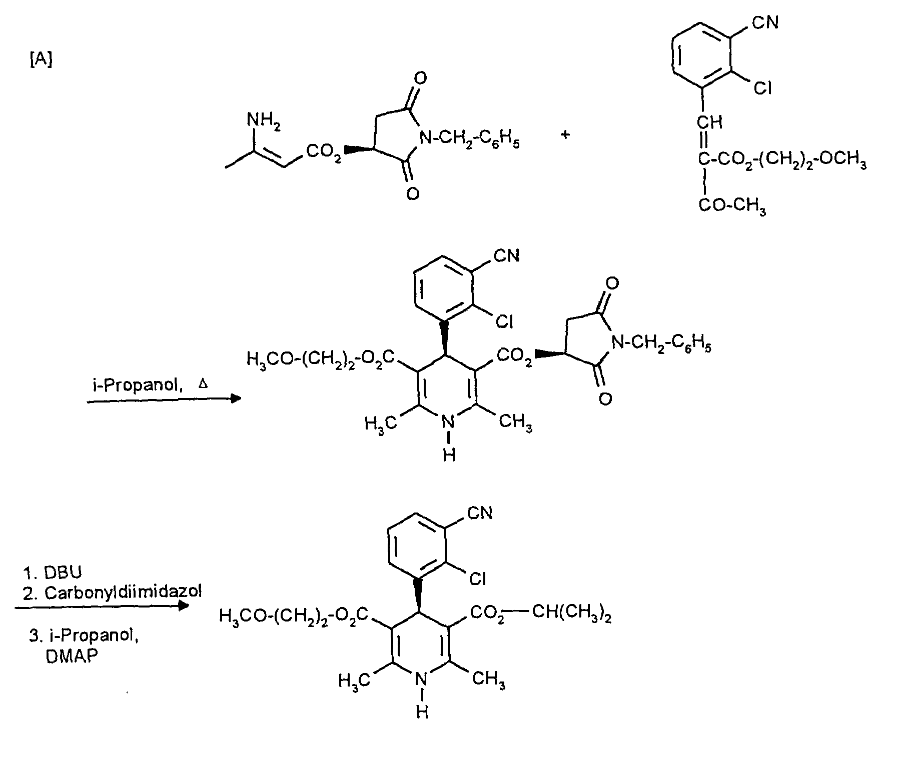 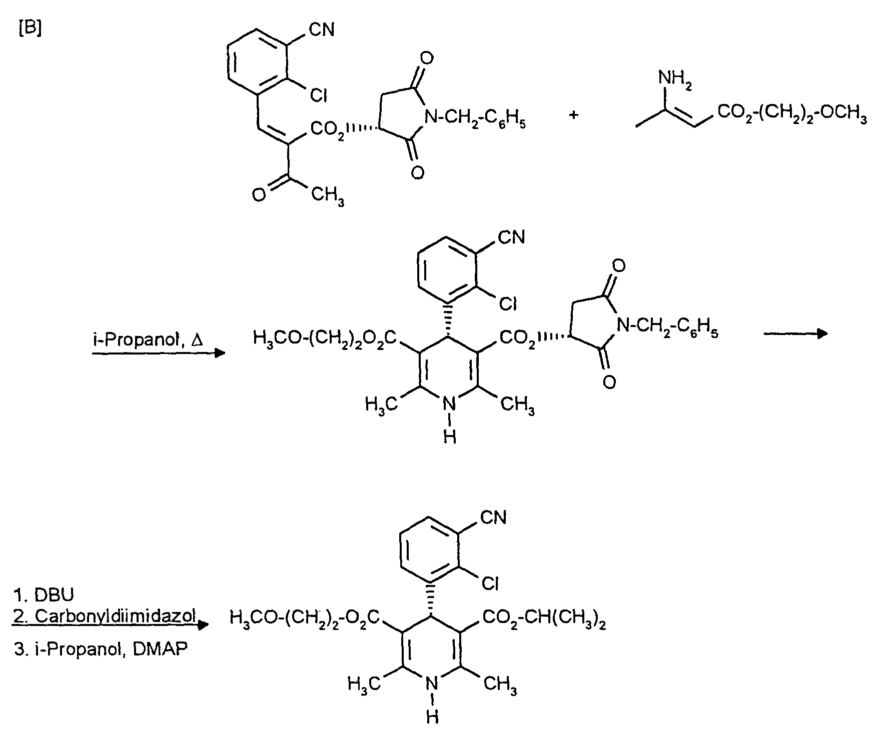 Überraschenderweise erhält man bei der Durchführung des erfindungsgemäßen Verfahrens die chiralen Verbindungen der allgemeinen Formel (I) auf elegante Weise mit sehr hoher Enantiomerenreinheit und gleichzeitig sehr guten Ausbeuten.

Im Gegensatz zum oben aufgeführten Stand der Technik ermöglicht das erfindungsgemäße Verfahren einen hochenantioselektiven Weg zur Synthese von enantiomerenreinen substituierten 4-Phenyl-1,4-dihydropyridin-3,5-dicarbonsäurederivaten durch Einsatz von Äpfelsäureimid-Resten in den Verbindungen der allgemeinen Formeln (IV) und (IVa) als Auxiliare, wobei diese in beiden enantiomerenreinen Formen vorliegen. Die Äpfelsäureimide wiederum sind über einen einzigen, chemisch leicht durchführbaren Reaktionsschritt aus der entsprechenden (R)- oder (S)-Äpfelsäure erhältlich. Das erfindungsgemäße Verfahren zeichnet sich außerdem dadurch aus, daß sich im Gegensatz zum Stand der Technik die Äpfelsäureimide als Auxiliare leicht in die Benzylidenverbindungen der allgemeinen Formel (II) oder in die Aminocrotonsäureester der allgemeinen Formel (IIIa) eingeführt werden können. Außerdem können die Äpfelsäureimid-Reste auf hochelegante Weise unter sehr milden Bedingungen mit schwachen Basen selektiv aus allen Verbindungen abgespalten werden. Darüber hinaus ist durch eine einfache und systematische Variation des Restes A in den Äpfelsäureimiden der allgemeinen Formeln (IV) und (IVa) eine auf das jeweilige Dihydropyridin optimale Problemlösung möglich. Aufgrund der rigiden, cyclischen Struktur der Äpfelsäureimide kristallisieren die entsprechenden diastereomerenreinen Dihydropyridine der allgemeinen Formel (IV/ IVa) zum einen in sehr guten Ausbeuten und zeichnen sich außerdem durch erheblich unterschiedliche Kristallisationsverhalten aus.

Diese Vorzüge, die letztendlich auch die sehr hohen Ausbeuten der erfindungsgemäßen Verbindungen der allgemeinen Formel (I) ermöglichen, werden durch kein bekanntes Auxiliar erreicht.

Ein weiterer Vorteil des erfindungsgemäßen Verfahrens liegt, insbesondere auch im Hinblick auf den Kostenfaktor, darin, daß die gesamte Reaktionssequenz sehr kurz und wenig aufwendig ist und bereits die verschiedenen Zwischenstufen in sehr guten Ausbeuten und mit hoher Diastereomeren- bzw. Enantiomerenreinheit erhalten werden können.

Das erfindungsgemäße Verfahren ist prinzipiell zur Synthese enantiomerenreiner Dihydropyridin-3,5-dicarbonsäurederivate geeignet.

Als Lösemittel für die Umsetzung der Verbindungen der Formeln (IIa) und (IIIa) eignen sich im allgemeinen alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure, oder Ester wie Ethylacetat, oder halogenierte Kohlenwasserstoffe wie Methylenchlorid, Tetrachlorkohlenstoff oder Kohlenwasserstoffe wie Benzol, Xylol oder Toluol. Ebenso ist es möglich, Gemische der genannten Lösemittel zu verwenden. Bevorzugt ist Isopropanol.

Die Reaktionstemperaturen können in einem größeren Breich variiert werden. Im allgemeinen arbeitet man zwischen 20°C und 120°C, vorzugsweise zwischen 60°C und 90°C.

Die Umsetzungen können bei Normaldruck, aber auch bei erhöhtem oder erniedrigtem Druck (z.B. 0,5 bis 80 bar) durchgeführt werden. Im allgemeinen arbeitet man bei Normaldruck.

Als Lösemittel für die Umsetzung der Verbindungen der Formel (II) und (III) eignen sich Ethylacetat oder Isopropanol.

Die Verbindungen der allgemeinen Formel (IIa) sind teilweise bekannt oder können nach üblichen Methoden, beispielsweise durch Umsetzung der entsprechenden Aldehyde mit Acetessigsäure-2-alkoxyalkylestern hergestellt werden.

Die Verbindungen der allgemeinen Formel (III) sind an sich bekannt.

Die enantiomerenreinen Benzylidenverbindungen der allgemeinen Formeln (II) sind neu und können hergestellt werden, indem man

Aldehyde der allgemeinen Formel (V)

R²-CHO (V)

in welcher
R² die oben angegebene Bedeutung hat,
mit enantiomerenreinen Acetessigsäureestern der allgemeinen Formel (VI) 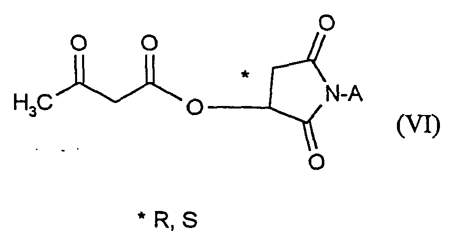 in welcher
A die oben angegebene Bedeutung hat,
in inerten Lösemitteln und in Anwesenheit einer Base und einer Carbonsäure
umsetzt.

Als Lösemittel für den ersten Schritt eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure, oder Ester wie Ethylacetat, oder halogenierte Kohlenwasserstoffe wie Dichlormethan, Tetrachlorkohlenstoff oder Kohlenwasserstoffe wie Benzol oder Toluol. Ebenso ist es möglich, Gemische der genannten Lösemittel zu verwenden. Bevorzugt ist Dichlormethan.

Als Basen für den ersten Schritt eignen sich vorzugsweise cyclische Amine, wie beispielsweise Piperidin, C₁-C₃-Tri- und Dialkylamine, wie beispielsweise Di- und Triethylamin oder Pyridin oder Dimethylaminopyridin. Bevorzugt ist Piperidin.

Die Base wird im allgemeinen in einer Menge von 0,01 mol bis 0,10 mol, bevorzugt von 0,05 mol bis 0,08 mol bezogen auf 1 mol des Aldehyds eingesetzt.

Als Säuren eignen sich vorzugsweise C₁-C₄-Alkylcarbonsäuren wie beispielsweise Essigsäuren.

Die Säure wird im allgemeinen in einer Menge von 0,01 mol bis 0,10 mol, bevorzugt von 0,05 mol bis 0,08 mol bezogen auf 1 mol des Aldehyds eingesetzt.

Die Reaktionstemperatur für den ersten Schritt kann in einem größeren Bereich variiert werden. Im allgemeinen arbeitet man in einem Bereich von 20°C bis 120°C, bevorzugt von 30°C bis 60°C.

Die Verfahren können bei Normaldruck, erhöhtem oder erniedrigtem Druck (beispielsweise von 0,5 bis 5 bar), vorzugsweise bei Normaldruck durchgeführt werden.

Die Aldehyde der allgemeinen Formel (V) sind bekannt oder können nach üblichen Methoden hergestellt werden.

Die enantiomerenreinen Verbindungen der allgemeinen Formel (VI) sind neu und können hergestellt werden, indem man
(S)- oder (R)-Äpfelsäureimide der allgemeinen Formel (VII) 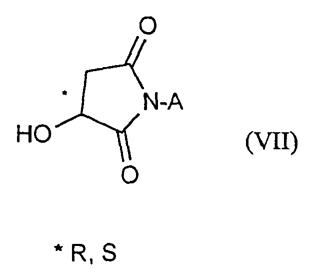 in welcher
A die oben angegebene Bedeutung hat,
mit Diketen oder Diketen-Aceton-Addukt (2,2,6-Trimethyl-1,3-dioxin-4-on) in inerten Lösemitteln umsetzt.

Als Lösemittel eignen sich im allgemeinen Kohlenwasserstoffe, wie beispielsweise Benzol, Toluol oder Xylol. Bevorzugt ist Toluol.

Die Umsetzungen erfolgen in einem Temperaturbereich von 90°C bis 140°C, bevorzugt von 100°C bis 110°C.

Die Umsetzungen werden im allgemeinen bei Normaldruck durchgeführt. Es ist aber auch möglich bei Über- oder Unterdruck (z.B. in einem Bereich von 0,5 bis 5 bar) zu arbeiten.

Die enantiomerenreinen Imide der allgemeinen Formel (VII) sind teilweise bekannt [vgl. z.B. THL 1990, 4949; J. Am. Chem. Soc., 2589, 1989] oder können hergestellt werden, indem man (S)-(-)- oder (R)-(-)-Äpfelsäure mit den entsprechenden Aminen, in einem der oben aufgeführten Lösemittel, vorzugsweise Xylol, in einem Temperaturbereich von 100°C bis 180°C, bevorzugt 130°C bis 150°C umsetzt.

Diketen und 2,2,6-Triethyl-1,3-dioxin-4-on sind bekannt.

Die enantiomerenreinen Aminocrotonsäureester der allgemeinen Formel (IIIa) sind neu und können beispielsweise hergestellt werden, indem man bei der Herstellung der oben aufgeführten Acetessigsäureester der allgemeinen Formel (VI) in situ Ammoniak oder Ammoniumsalze zugibt.

Als Lösemittel eignen sich die für die Herstellung der Verbindungen der allgemeinen Formel (VI) genannten. Die Umsetzung mit den Ammoniumsalzen erfolgt in Toluol am Wasserabscheider unter Rückfluß.

Die Umsetzungen erfolgen in einem Temperaturbereich von 50°C bis 120°C, bevorzugt von 5°C bis 80C.

Die Umsetzungen werden im allgemeinen bei einem Unterdruck von 0,1 bis 0,5 bar durchgeführt. Es ist aber auch möglich bei Normal- oder Überdruck (z.B. in einem Bereich von 1 bis 5 bar) zu arbeiten.

Als Ammoniumsalze eignen sich im allgemeinen Ammoniumsalze organischer oder anorganischer Säuren, wie beispielsweise Ammoniumacetat oder Ammoniumformiat. Bevorzugt ist Ammoniumacetat.

Die enantiomerenreinen Verbindungen der allgemeinen Formel (IV) sind neu und können wie oben beschrieben hergestellt werden.

Die Abspaltung des substituierten Pyrrolidin-2,5-dion-3-yl-Restes aus den enantiomerenreinen 1,4-Dihydropyridinen der allgemeinen Formel (IV) erfolgt in einem der oben aufgeführten inerten Lösemittel. Bevorzugt sind Ethylacetat, Tetrahydrofuran oder Gemische der beiden.

Als Basen eignen sich im allgemeinen Alkalicarbonate wie beispielsweise Natrium- oder Kaliumcarbonat, oder organische Basen wie Trialkylamine, z.B. Triethylamin, N-Ethylmorpholin, N-Methylpiperidin oder Diisopropylethylamin, oder Dimethylaminopyridin, 1,8-Diazabicyclo[5.4.0]undec-7-en (DBU) oder 1,5-Diazabicyclo[4.3.0]non-5-en (DBN). Bevorzugt ist 1,8-Diazabicyclo[5.4.0]undec-7-en.

Die Base wird in einer Menge von 1 mol bis 5 mol, bevorzugt von 1 mol bis 2 mol jeweils bezogen auf 1 mol der enantiomerenreinen Verbindungen der allgemeinen Formel (IV) eingesetzt.

Die Umsetzung erfolgt in einem Temperaturbereich von 0°C bis 50°C, bevorzugt bei Raumtemperatur.

Die Umsetzung wird im allgemeinen bei Normaldruck durchgeführt. Es ist aber auch möglich bei Über- oder Unterdruck (z.B. in einem Bereich von 0,5 bis 5 bar) zu arbeiten.

Ohne Isolierung der freien enantiomerenreinen Säure werden die Verbindungen der allgemeinen Formeln (IV) oder (IVa) anschließend durch Aktivierung mit einem Hilfsmittel, in einem der oben aufgeführten Lösemittel in Anwesenheit von Ethylacetat in die enantiomerenreinen Verbindungen der allgemeinen Formel (VIII) 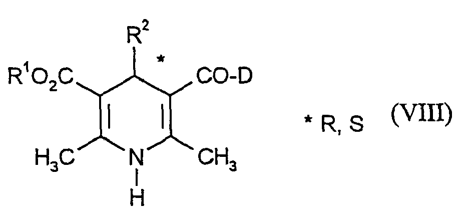 in welcher
- R¹ und R²: die oben angegebene Bedeutung haben,
und
- D: für einen aktivierenden Rest, beispielsweise für Imidazolyl steht,
überführt, und in einem letzten Schritt mit dem entsprechenden Alkohol (R³-OH), in Anwesenheit einer der oben aufgeführten Basen, vorzugsweise N,N-Dimethylaminopyridin, bei der jeweiligen Rückflußtemperatur des Alkohols, zu den erfindungsgemäßen enantiomerenreinen Verbindungen der allgemeinen Formel (I) umgesetzt.

Als Hilfsstoffe zur Aktivierung der Carbonsäure werden bevorzugt Kondensationsmittel eingesetzt. Bevorzugt werden hier die üblichen Kondensationsmittel wie Carbodiimide z.B. N,N'-Diethyl-, N,N'-Dipropyl-, N,N'-Diisopropyl-, N,N'-Dicyclohexylcarbodiimid, N-(3-Dimethylaminoisopropyl)-N'-ethylcarbodiimid-Hydrochlorid, oder Carbonylverbindungen wie Carbonyldiimidazol, oder 1,2-Oxazoliumverbindungen wie 2-Ethyl-5-phenyl-1,2-oxazolium-3-sulfonat oder 2-tert.Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris(dimethylamino)phosphonium-hexafluorophosphonat eingesetzt. Bevorzugt sind N,N'-Dicyclohexylcarbodiimid und Carbonyldiimidazol.

Die Hilfsstoffe werden im allgemeinen in einer Menge von 1 mol bis 3 mol, bevorzugt von 1 mol bis 1,5 mol, jeweils bezogen auf 1 mol der freien Carbonsäure eingesetzt.

Die Verfahren können bei Normaldruck, erhöhtem oder erniedrigtem Druck (beispielsweise von 0,5 bis 5 bar), vorzugsweise bei Normaldruck durchgeführt werden.

Die aktivierten enantiomerenreinen 1,4-Dihydropyridine der allgemeinen Formel (VIII) sind bekannt oder können wie oben beschrieben hergestellt werden.

Bevorzugt werden nach dem erfindungsgemäßen Verfahren enantiomerenreine Verbindungen der allgemeinen Formel (I),
in welcher
- R¹ und R³: gleich oder verschieden sind und
für geradkettiges oder verzweigtes Alkyl mit bis zu 8 Kohlenstoffatomen stehen, das gegebenenfalls durch geradkettiges oder verzweigtes Alkoxy mit bis zu 5 Kohlenstoffatomen oder Hydroxy substituiert ist, oder für Cyclopropyl, Cyclopentyl, Cyclohexyl oder Cycloheptyl stehen,
und
- R²: für den Rest 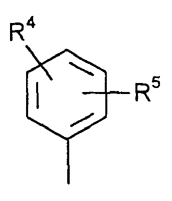 steht,
worin
R⁴ und R⁵ gleich oder verschieden sind und jeweils Fluor, Brom, Chlor, Cyano, Ethinyl, Trifluormethoxy, Methyl, Nitro, Methylthio, Trifluormethyl oder geradkettiges oder verzweigtes Alkoxy mit bis zu 3 Kohlenstoffatomen bedeuten, und gegebenenfalls einer der Substituenten für Wasserstoff steht,
und deren Salze.

Besonders bevorzugt werden nach dem erfindungsgemäßen Verfahren Verbindungen der allgemeinen Formel (I),
in welcher
- R¹ und R³: gleich oder verschieden sind und
für geradkettiges oder verzweigtes Alkyl mit bis zu 8 Kohlenstoffatomen stehen, das gegebenenfalls durch Methoxy oder Hydroxy substituiert ist, oder
für Cyclopropyl, Cyclopentyl, Cyclohexyl oder Cycloheptyl stehen,
und
- R²: für den Rest 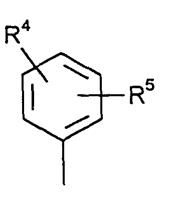 steht,
worin
R⁴ und R⁵ gleich oder verschieden sind und Fluor, Chlor, Cyano, Ethinyl, Trifluormethoxy, Methyl, Methylthio, Nitro, Trifluormethyl oder geradkettiges oder verzweigtes Alkoxy mit bis zu 3 Kohlenstoffatomen bedeuten, und gegebenenfalls einer der Substituenten für Wasserstoff steht,
und deren Salze.

Ganz besonders bevorzugt nach dem erfindungsgemäßen Verfahren werden die enantiomerenreinen Verbindungen (4R)- und (4S)-Isopropyl-(2-methoxyethyl)-4-(2-chlor-3-cyano-phenyl)-1,4-dihydro-2,6-dimethyl-pyridin-3,5-dicarboxylat hergestellt.

Das erfindungsgemäße Verfahren ermöglicht auf hochenantioselektive Weise und gleichzeitig sehr hoher Ausbeute den Zugang zu den enantiomerenreinen Halogenphenyl-substituierten 1,4-Dihydropyridinen der allgemeinen Formel (I), die wertvolle cerebral wirksame Arzneimittel darstellen.

### Ausgangsverbindungen

### Beispiel I (Formel VI)

### (3S)-1-Benzyl-3-(3-oxobutyryloxy)-pyrrolidin-2,5-dion

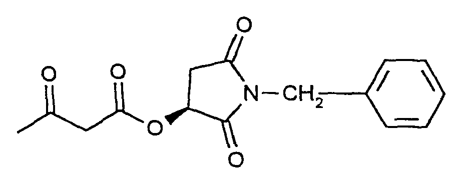

Zu einer Lösung von (S)-Äpfelsäure-N-benzylimid (9,0 g, 43,8 mmol) [THL 1990, 4949] in Xylol (18 ml) tropft man bei 130°C 2,2,6-Trimethyl-1,3-dioxin-4-on (6,6 g, 4,38 mmol, 95%ig). Das entstehende Aceton wird aus dem Reaktionsgemisch abdestilliert. Man läßt noch 2 h bei 130°C rühren, kühlt die Reaktionslösung auf 50°C und zieht das Lösemittel im Vakuum ab. Der Rückstand wird säulenchromatographisch an Kieselgel gereinigt (Laufmittel: Diethylether). Nach Einengen der Produktfraktionen erhält man 11,8 g (93%) der Titelverbindung.
¹H-NMR (CDCl₃):δ = 2,28 (s, 3H); 2,77 (dd, J = 18 Hz, 5 Hz, 1H); 3,19 (dd, J = 18 Hz, 8 Hz, 1H); 3,56 (s, 2H); 4,68 (AB-System, 2H); 5,49 (dd, J = 8 Hz, 5 Hz, 1H); 7,25 - 7,42 ppm (m, 5H); Enol-H: schwaches Singulett bei 11,68 ppm).

### Beispiel II

### (3S)-3-(3-Aminocrotonyloxy)-1-benzyl-pyrrolidin-2,5-dion

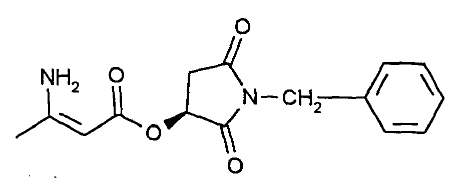

Man erhitzt eine Suspension von (S)-Äpfelsäure-N-benzylimid (1700 g, 8,28 mol) in Toluol (6,8 1) auf 105°C und läßt innerhalb von ca. 20 Min. 2,2,6-Trimethyl-1,3-dioxin-4-on (85%ig, 1447 g, 8,65 mol) zulaufen, wobei das entstehende Aceton zusammen mit Toluol abdestilliert. Man läßt noch 2 h bei 100 - 105°C rühren, wobei weiter Aceton / Toluol abdestilliert wird. Man läßt Toluol (1 1) zur Reaktionslösung laufen und kühlt den Ansatz auf 70°C ab. Nach Zugabe von Ammoniumacetat (1207 g, 15,7 mol) wird 3 h bei 65°C und 250 - 300 mbar am Wasserabscheider unter Rückfluß erhitzt. Man läßt Ethylacetat (3,4 1) zulaufen, kühlt den Ansatz auf Raumtemperatur ab, wäscht mit ges. wäßriger NaHCO₃₋Lösung, trocknet die organische Phase über Na₂SO₄, und destilliert das Lösemittel im Vakuum bei 35-40°C ab. Der Rückstand wird in Isopropanol (4,2 1) aufgenommen, und das Lösemittel wird im Vakuum bei 25-65°C abdestilliert. Der Rückstand wird erneut in Isopropanol (2,5 1) aufgenommen. Man erhitzt die Suspension zum Rückfluß, wobei der Feststoff in Lösung geht. Nach dem Abkühlen auf 5-7°C läßt man Wasser (1,8 1) zulaufen, filtriert ausgefallenes Produkt ab, wäscht mit Isopropanol / Wasser (1:1, 3,4 1) und trocknet das Produkt im Vakuum bei 50°C.
Ausbeute: 1990 g (83%)
Schmp.: 104-105°C
¹H-NMR (CDCl₃) δ = 1,94 (s, 3H); 2,71 (dd, J = 18 Hz, 5 Hz, 1H); 3,12 (dd, J = 18 Hz, 8 Hz, 1H); 4,57 (s, 1H); 4,71 (AB-System, 2H); 4,74 (s, breit, 1H); 5,40 (dd, J = 8 Hz, 5 Hz, 1H); 7,20 - 7,44 (m, 5H); 7,88 ppm (s, breit, 1H).

### Beispiel III (Formel II)

### (3'S)-2-Acetyl-3-(2-chlor-3-cyanophenyl)-2-propensäure-(1-benzylpyrrolidin-2,5-dion-3-yl)ester

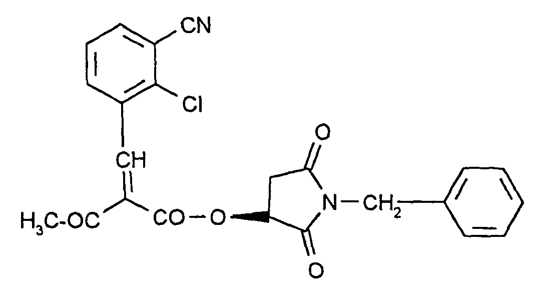

Eine Lösung der Verbindung aus Beispiel I (12,6 g, 43,6 mmol) und 2-Chlor-3-cyano-benzaldehyd (7,2 g, 43,6 mmol) in Dichlormethan (80 ml) wird mit Piperidin (246 mg, 2,8 mmol) und Eisessig (168 mg, 2,8 mmol) versetzt und 18 h am Wasserabscheider unter Rückfluß erhitzt. Nach Abkühlen auf Raumtemperatur wird die Dichlormethan-Lösung mit Wasser (40 ml) gewaschen, über Na₂SO₄ getrocknet und im Vakuum eingeengt. Der Rückstand wird säulenchromatographisch an Kieselgel gereinigt (Lauftmittel: Ether). Nach Einengen der Produktfraktionen erhält man 13,0 g (68%) der Titelverbindung als Gemisch von E/Z-Isomeren.
¹H-NMR (CDCl₃): δ = 2,30, 2,51 (2s, 3H); 2,70 - 2,87 (m, 1H); 3,08 - 3,33 (m, 1H); 4,63 - 4,80 (m, 2H); 5,51 - 5,69 (m, 1H); 7,27 - 7,92 (m, 9H).

### Beispiel IV (Formel IV)

### (4R,3' S)-(1-Benzyl-pyrrolidin-2,5-dion-3-yl)-(2-methoxyethyl)-4-(2-chlor-3-cyanophenyl)-1,4-dihydro-2,6-dimethyl-pyridin-3,5-dicarboxylat

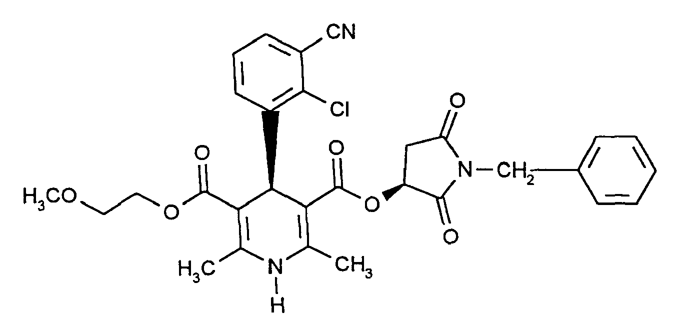

### Variante A:

Die Verbindung aus Beispiel II (80,0 g, 0,244 mol) und 2-Acetyl-3-(2-chlor-3-cyano-phenyl)-2-propensäure-2-methoxyethylester (83,29 g, 0,243 mol) werden mit Isopropanol (1100 ml) versetzt und die Mischung wird 8,5 h unter Rückfluß erhitzt. Man kühlt auf Raumtemperatur ab, filtriert das ausgefallene Rohprodukt ab, wäscht zweimal mit je 100 ml Isopropanol und trocknet das Rohprodukt im Vakuum bei 40°C. Das Rohprodukt wird in Ethylacetat (200 ml) suspendiert und die Suspension wird 1 h unter Rückfluß erhitzt. Nach dem Abkühlen auf Raumtemperatur filtriert man das Produkt ab, wäscht mit Ethylacetat (40 ml) und trocknet das Produkt im Vakuum bei 50°C.
Ausbeute: 57,8 g (41%)
Diastereomerenüberschuß ≥ 99,5% (HPLC, Chiracel OD-H)
Schmp.: 239-240°C
¹H-NMR (d₆-DMSO): δ = 2,26 (s, 6H); 2,68 (dd, J = 18 Hz, 5 Hz, 1H); 3,09 (dd, J = 18 Hz, 8 Hz, 1H); 3,16 (s, 3H); 3,37 - 3,50 (m, 2H); 3,95 - 4,12 (m, 2H); 4,52, 4,64 (AB-Signal, J_{AB} = 15 HZ, 2H); 5,25 (s, 1H); 5,53 (dd, J = 8 Hz, 5 Hz, 1H); 7,22 - 7,77 (m, 8H).

### Variante B (über II und III):

Die Verbindung aus Beispiel III (3,0 g, 6,9 mmol) und 3-Aminocrotonsäure-(2-methoxyethyl)-ester (1,1 g, 6,9 mmol) werden mit Ethylacetat (38 ml) versetzt und 5 h mit Rückfluß erhitzt. Man filtriert ausgefallenes Produkt ab, wäscht mit Ethylacetat (3 ml) und trocknet das Produkt im Vakuum bei 40°C.
Ausbeute: 1,3 g (33%)
Diastereomerenüberschuß: ≥ 99,5% (HPLC, Chiracel OD-H)

### Beispiel V

### (4R)-Imidazolyl-(2-methoxyethyl)-4-(2-chlor-3-cyano-phenyl)-1,4-dihydro-2,6-dimethyl-pyridin-3,5-dicarboxylat

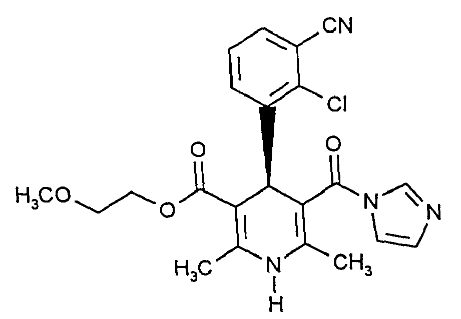

Die Verbindung aus Beispiel IV (73,9 g, 0,128 mol) wird in Ethylacetat (480 ml) und Tetrahydrofuran (96 ml) suspendiert, mit 1,8-Diazabicyclo[5.4.0]undec-7-en (DBU, 29,0 ml, 0,192 mol) versetzt und 12 h bei Raumtemperatur gerührt. Anschließend wird 1 N HCl (300 ml) zugegeben und 15 Minuten kräftig aufgerührt. Die Ethylacetat-Phase wird abgetrennt, je einmal mit 1 N HCl (150 ml) und ges. wäßriger NaCl-Lösung gewaschen und über Na₂SO₄ getrocknet. Das Lösemittel wird im Vakuum abgezogen und der Rückstand mit Ethylacetat (420 ml) aufgeommen. Nach Zugabe von N.N'-Carbonyldiimidazol (25,0 g, 0,154 mol) wird 12 h bei Raumtemperatur und 30 Minuten bei 0-5°C gerührt. Ausgefallenes Produkt wird abfiltriert, mit Ethylacetat (25 ml) gewaschen und im Vakuum getrocknet.
Ausbeute: 42,6 g (76%)
Schmp.: 180°C
¹H-NMR (CDCl₃): δ = 1,90 (s, 3H); 2,48 (s, 3H); 3,22 (s, 3H); 3,40 - 3,52 (m, 2H); 4,10 (t, 2H); 5,58 (s, 1H); 6,02 (s, 1H); 7,08 (d, 1H); 7,25 - 7,58 (m, 4H); 7,91 (s, 1H).

### Dihydropyridine der Formel (I)

### Beispiel 1

### (4R)-Isopropyl-(2-methoxyethyl)-4-(2-chlor-3-cyano-phenyl)-1,4-dihydro-2,6-dimethyl-pyridin-3,5-dicarboxylat

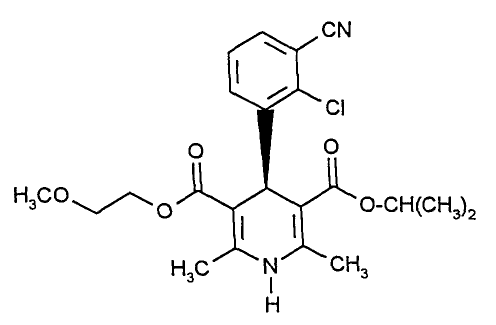

Die Verbindung aus Beispiel V (73,2 g, 166 mmol) und N,N-Dimethylaminopyridin (0,93 g, 7,6 mmol) werden in Isopropanol (530 ml) 20 h unter Rückfluß erhitzt. Die Reaktionsmischung wird langsam auf 0-5°C abgekühlt und 1 h bei 0-5°C gerührt. Auskristallisiertes Rohprodukt wird abfiltriert, mit kaltem Isopropanol (35 ml) gewaschen und im Vakuum getrocknet. Nach Umkristallisation des Rohproduktes aus Ethylacetat (150 ml) / Cyclohexan (450 ml) erhält man 53,2 g (74%) der Titelverbindung.
Schmp.: 138-140°C
[α]²⁰_{D} = +13,9 (c = 1, CHCl₃)

## Patentansprüche

1. Verfahren zur Herstellung von enantiomerenreinen phenylsubstituierten 1,4-Dihydropyridin-3,5-dicarbonsäurederivaten der allgemeinen Formel (I) 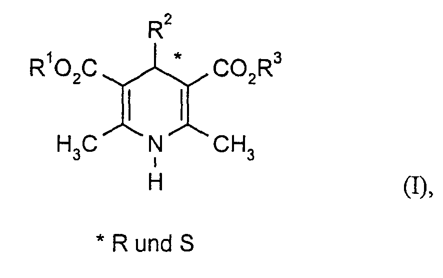 in welcher
R¹ und R³ gleich oder verschieden sind und für geradkettiges oder verzweigtes Alkyl mit bis zu 8 Kohlenstoffatomen stehen, das gegebenenfalls durch geradkettiges oder verzweigtes Alkoxy mit bis zu 6 Kohlenstoffatomen oder Hydroxy substituiert ist, oder
für Cycloalkyl mit 3 bis 7 Kohlenstoffatomen stehen,
und
R² für den Rest 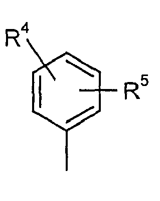 steht,
worin
R⁴ und R⁵ gleich oder verschieden sind und Halogen, Cyano, Ethinyl, Trifluormethoxy, Methylthio, Nitro, Trifluormethyl oder geradkettiges oder verzweigtes Alkyl, Alkenyl, Alkinyl oder Alkoxy bis zu 4 Kohlenstoffatomen bedeuten, und gegebenenfalls einer der Substituenten für Wasserstoff steht, und deren Salze,
**dadurch gekennzeichnet, dass** man die enantiomerenreinen Benzylidenverbindungen der allgemeinen Formel (II) oder die Benzylidenverbindungen der allgemeinen Formel (IIa), 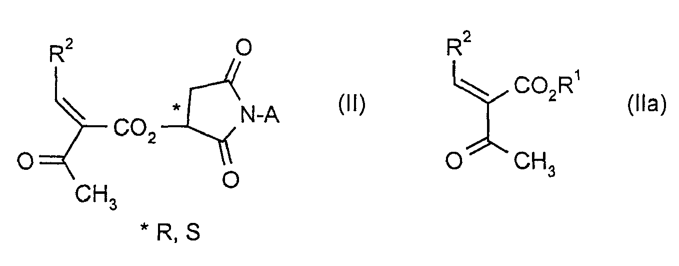 in welchen
R¹ und R² die oben angegebenen Bedeutungen haben
und
A für Wasserstoff oder für geradkettiges oder verzweigtes Alkyl mit bis zu 8 Kohlenstoffatomem steht, oder
für Phenyl oder Benzyl steht, die gegebenenfalls bis zu 3-fach gleich oder verschieden durch Hydroxy, Nitro, Halogen, Cyano, Carboxy, Trifluormethyl, Trifluormethoxy, geradkettiges oder verzweigtes Alkoxy mit bis zu 6 Kohlenstoffatomen oder durch eine Gruppe der Formel -NR⁶R⁷ oder -SO₂R⁸ substituiert sind,
worin
R⁶ und R⁷ gleich oder verschieden sind und Wasserstoff, Phenyl oder geradkettiges oder verzweigtes Alkyl mit bis zu 5 Kohlenstoffatomen bedeuten
und
R⁸ geradkettiges oder verzweigtes Alkyl mit bis zu 4 Kohlenstoffatomen oder Phenyl bedeutet,
durch Umsetzung im Fall der enantiomerenreinen Benzylidenverbindungen der allgemeinen Formel (II) mit Aminocrotonsäureestern der allgemeinen Formel (III) und im Fall der Benzylidenverbindungen der allgemeinen Formel (IIa) mit den entsprechenden enantiomerenreinen Aminocrotonsäureestern der allgemeinen Formel (IIIa), 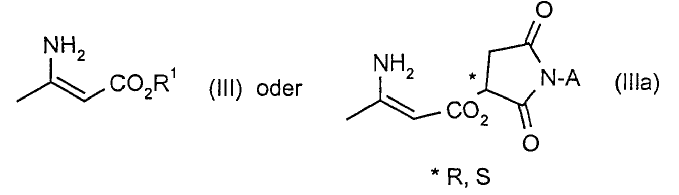
in welchen
R¹ und A die in Anspruch 1 angegebenen Bedeutungen haben,
in inerten Lösemitteln, gegebenenfalls in Anwesenheit einer Base,
in die diastereomerenreinen 1,4-Dihydropyridine der allgemeinen Formeln (IVa) und (IVb) 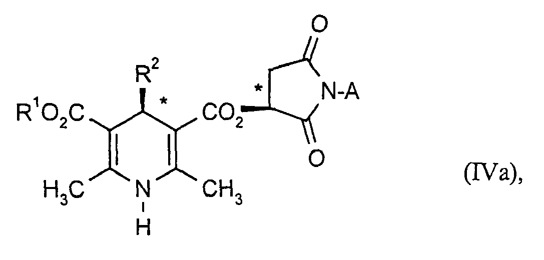 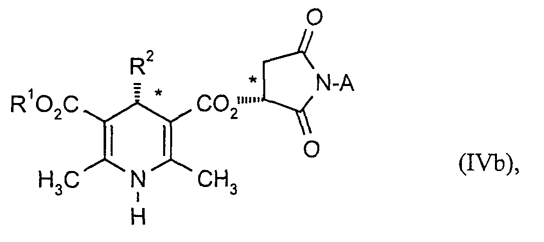 in welchen
R¹, R² und A die oben angegebenen Bedeutungen haben, überführt,
und anschließend den Äpfelsäureimid-Rest unter milden Bedingungen mit schwachen Basen, gegebenenfalls unter Isolierung der freien Säure, abspaltet, und die Carbonsäurefunktion nach üblichen Methoden verestert.

2. 1,4-Dihydropyridine der allgemeinen Formeln (IVa) und (IVb) gemäß Anspruch 1.

## Claims

1. Process for the preparation of enantiomerically pure phenyl-substituted 1,4-dihydropyridine-3,5-dicarboxylic acid derivatives of the general formula (I) 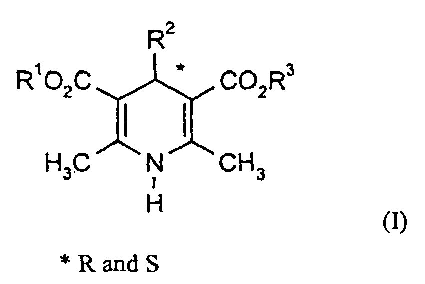 in which
R¹ and R³ are identical or different and represent straight-chain or branched alkyl having up to 8 carbon atoms which is optionally substituted by straight-chain or branched alkoxy having up to 6 carbon atoms or hydroxyl, or
represent cycloalkyl having 3 to 7 carbon atoms,
and
R² represents the radical 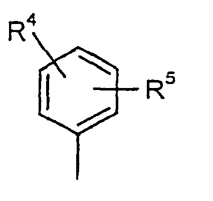
in which
R⁴ and R⁵ are identical or different and denote halogen, cyano, ethinyl, trifluoromethoxy, methylthio, nitro, trifluoromethyl or straight-chain or branched alkyl, alkenyl, alkinyl or alkoxy having up to 4 carbon atoms, and one of the substituents optionally represents hydrogen,
and their salts,
**characterized in that** the enantiomerically pure benzylidene compounds of the general formula (II) or the benzylidene compounds of the general formula (IIa) 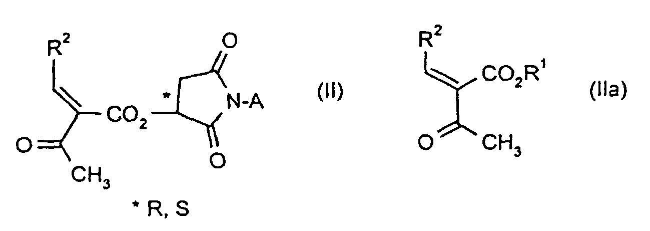 in which
R¹ and R² have the abovementioned meanings
and
A represents hydrogen or straight-chain or branched alkyl having up to 8 carbon atoms, or
represents phenyl or benzyl which are optionally up to trisubstituted by identical or different substituents from the series consisting of hydroxyl, nitro, halogen, cyano, carboxyl, trifluoromethyl, trifluoromethoxy, straight-chain or branched alkoxy having up to 6 carbon atoms or by a group of the formula -NR⁶R⁷ or -SO₂R⁸
in which
R⁶ and R⁷ are identical or different and denote hydrogen, phenyl or straight-chain or branched alkyl having up to 5 carbon atoms
and
R⁸ denotes straight-chain or branched alkyl having up to 4 carbon atoms or phenyl,
are converted by reaction
in the case of the enantiomerically pure benzylidene compounds of the general formula (II), with aminocrotonic esters of the general formula (III) and, in the case of the benzylidene compounds of the general formula (IIa), with the corresponding enantiomerically pure aminocrotonic esters of the general formula (IIIa) 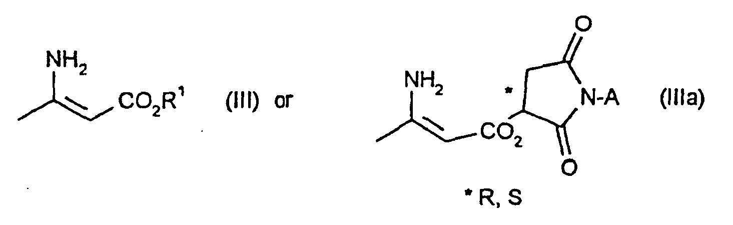 in which
R¹ and A have the meanings given in Claim 1
in inert solvents, if appropriate in the presence of a base,
to the diastereomerically pure 1,4-dihydropyridines of the general formulae (IVa) and (IVb) 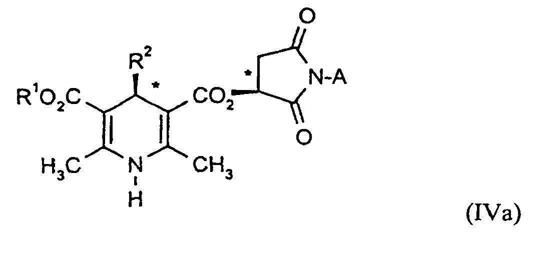 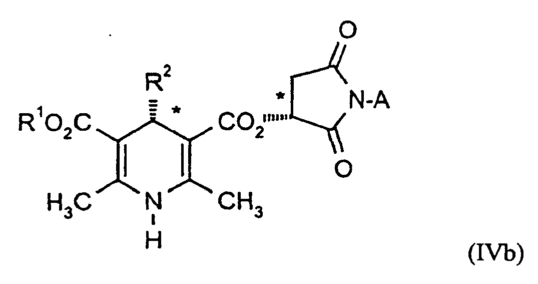 in which
R¹, R² and A have the abovementioned meanings
and the maleimide radical is subsequently eliminated with weak bases under mild conditions, if appropriate isolating the free acid, and the carboxyl function is esterified by customary methods.

2. 1,4-Dihydropyridines of the general formulae (IVa) and (IVb) according to Claim 1.

## Revendications

1. Procédé de production de dérivés énantiomériquement purs d' acide 1,9-dihydropyridine-3, 5-dicarboxylique à substituant phényle de formule générale (I) 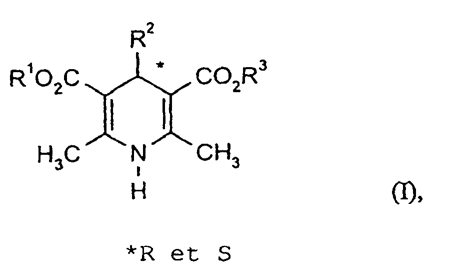 dans laquelle
R¹ et R³ sont identiques ou différents et représentent un reste alkyle linéaire ou ramifié ayant jusqu'à 8 atomes de carbone, qui est éventuellement substitué par un radical alkoxy linéaire ou ramifié ayant jusqu'à 6 atomes de carbone ou par un radical hydroxy, ou bien
un reste cycloalkyle ayant 3 à 7 atomes de carbone,
et
R² représente le reste 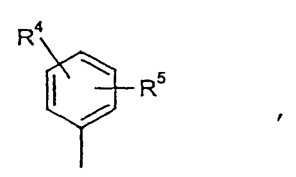 dans lequel
R⁴ et R⁵ sont identiques ou différents et représentent un halogène, un groupe cyano, un reste éthynyle, trifluorométhoxy, méthylthio, nitro, trifluorométhyle ou un reste alkyle, alcényle, alcynyle ou alkoxy linéaire ou ramifié ayant jusqu'à 4 atomes de carbone, et le cas échéant, l'un des substituants représente l'hydrogène, et un de leurs sels,
**caractérisé en ce qu'**on transforme les dérivés benzylidéniques énantiomériquement purs de formule générale (II) ou les composés benzylidéniques de formule générale (IIa), 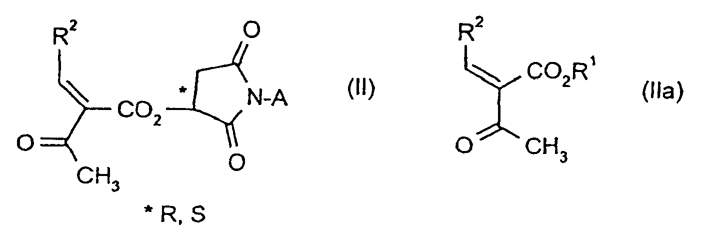 dans lesquels
R¹ et R² ont les définitions indiquées ci-dessus
et
A représente l'hydrogène ou un reste alkyle linéaire ou ramifié ayant jusqu'à 8 atomes de carbone, ou bien
un reste phényle ou un reste benzyle, qui sont éventuellement substitués jusqu'à 3 fois identiques ou différentes par un radical hydroxy, nitro, halogéno, cyano, carboxy, trifluorométhyle, trifluorométhoxy, alkoxy linéaire ou ramifié ayant jusqu'à 6 atomes de carbone ou par un groupe de formule -NR⁶R⁷ ou -SO₂R⁸,
dans laquelle
R⁶ et R⁷ sont identiques ou différents et représentent l'hydrogène, un reste phényle ou un reste alkyle linéaire ou ramifié ayant jusqu'à 5 atomes de carbone
et
R⁸ est un reste alkyle linéaire ou ramifié ayant jusqu'à 4 atomes de carbone ou un reste phényle,
par réaction, dans le cas des composés benzylidéniques énantiomériquement purs de formule générale (II), avec des esters d'acide aminocrotonique de formule générale (III), et dans le cas des composés benzylidéniques de formule générale (IIa), avec les esters d'acide aminocrotonique énantiomériquement purs correspondants de formule générale (IIIa), 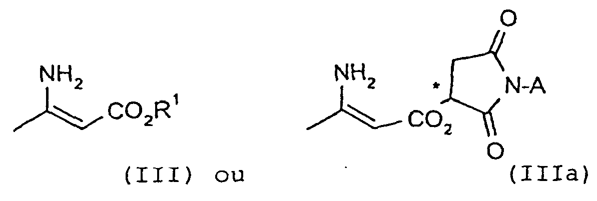 formules dans lesquelles
R¹ et A ont les définitions indiquées dans la revendication 1, dans des solvants inertes, éventuellement en présence d'une base,
en les 1,4-dihydropyridines diastéréo-isomériquement pures de formules générales (IVa) et (IVb) 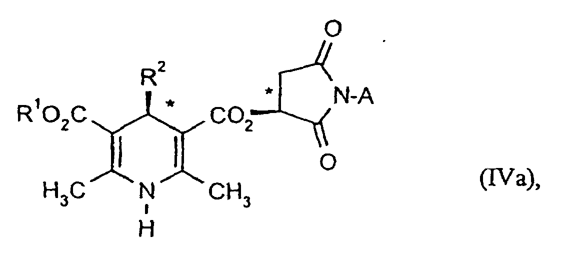 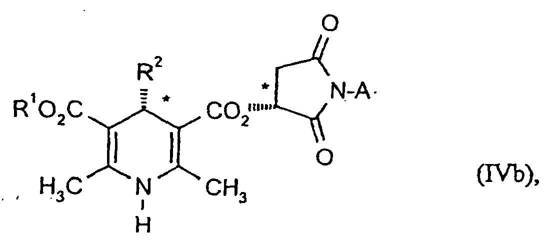 dans lesquelles
R¹, R² et A ont les définitions indiquées ci-dessus,
puis on élimine le reste imide d'acide malique dans des conditions douces avec des bases faibles, en isolant éventuellement l'acide libre, et on estérifie la fonction acide carboxylique selon des modes opératoires usuels.

2. 1,4-dihydropyridines de formules générales (IVa) et (IVb) selon la revendication 1.
